(19) Europäisches Patentamt
European Patent Office
Office européen des brevets

(11) EP 1 471 901 B1

(12) **EUROPEAN PATENT SPECIFICATION**

(45) Date of publication and mention of the grant of the patent:
**23.07.2008 Bulletin 2008/30**

(21) Application number: **03704764.4**

(22) Date of filing: **05.02.2003**

(51) Int Cl.:
***A61K 31/155*** *(2006.01)* ***A61K 31/16*** *(2006.01)*
***A61K 31/405*** *(2006.01)* ***A61P 25/28*** *(2006.01)*

(86) International application number:
**PCT/GB2003/000507**

(87) International publication number:
**WO 2003/066037 (14.08.2003 Gazette 2003/33)**

(54) **SPERMIDINE DERIVATIVE FOR THE TREATMENT OF CHRONIC NEURODEGENERATIVE DISEASES**

SPERMIDIN DERIVATE ZUR BEHANDLUNG CHRONISCHER NEURODEGERENATIVER KRANKHEITEN

DERIVE DE SPERMIDINE DESTINE AU TRAITEMENT DE MALADIES NEURODEGENERATIVES CHRONIQUES

(84) Designated Contracting States:
**AT BE BG CH CY CZ DE DK EE ES FI FR GB GR HU IE IT LI LU MC NL PT SE SI SK TR**

(30) Priority: **05.02.2002 GB 0202645**

(43) Date of publication of application:
**03.11.2004 Bulletin 2004/45**

(73) Proprietor: **UNIVERSITY OF SOUTHAMPTON**
**Southampton,**
**Hampshire SO17 1BJ (GB)**

(72) Inventors:
- **MORRISON, Barclay III**
  **London, SE1 7QU (GB)**
- **PRINGLE, Ashley, Ker**
  **Southampton,**
  **Hampshire SO19 7JF (GB)**
- **SUNDSTROM, Lars, Eric**
  **Old Alresford,**
  **Hampshire SO24 9DU (GB)**
- **WÜLFERT, Ernst**
  **B-1180 Brussels (BE)**

(74) Representative: **Tubby, David George**
**Marks & Clerk**
**90 Long Acre**
**London WC2E 9RA (GB)**

(56) References cited:
**WO-A-91/00853** **WO-A-93/12777**
**WO-A-99/03823** **WO-A-99/31049**

- **DATABASE CA [Online] CHEMICAL ABSTRACTS SERVICE, COLUMBUS, OHIO, US; MARSH, IAN R. ET AL: "Synthetic methods for polyamine linkers and their application to combinatorial chemistry" retrieved from STN Database accession no. 127:66130 XP002243815 & MOLECULAR DIVERSITY, vol. 2, no. 3, 1997, pages 165-170,**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to the treatment of chronic neurodegenerative diseases or conditions, such as Alzheimer's Disease, Parkinson's Disease, Huntington's Chorea and Multiple Sclerosis, using neuroprotective compounds. The invention also relates to the protection from or treatment of damage or diseases resulting from superoxide production or peroxynitrite production.

In tissues affected by low oxygen states such as prolonged hypoxia and ischaemia, which may or may not be associated with hypoglycaemia, neuronal damage, to varying degrees, is encountered. Ischaemia typically occurs as a result of an acute event, for example heart attack, stroke or traumatic head injury. During heart attack, the damage incurred is substantially limited to the heart tissues, and certain treatments have been developed. In stroke or traumatic head injury, neuronal damage results from the effects of more long term ischaemia on the brain. The severity of the ischaemia depends on the nature of the stroke or injury, but, invariably, there is brain damage. WO99/31049 addresses the effects of ischaemia on the brain such as occurs with stroke patients or as a result of head injury, and discloses certain neuroprotective agents and their use in treating neuronal damage caused by acute ischaemic events such as stroke and head injury.

In contrast to the neuronal damage occurring as a result of acute ischaemic events such as heart attack, stroke or head injury, the present invention addresses the effects of chronic neurodegenerative diseases or conditions, such as Alzheimer's Disease (AD), Parkinson's Disease (PD), Huntington's Chorea (HC), Multiple Sclerosis (MS) and Amyotrophic Lateral Sclerosis (ALS).

The underlying causes of these neurodegenerative diseases are complex and appear to be multifactorial. In each case, necrotic and apoptotic neuronal cell death may result from one or more mechanisms including metabolic compromise, excitotoxicity and oxidative stress. A number of studies point towards oxidative stress as a major causative factor in a variety of chronic neurodegenerative diseases including AD, PD and ALS (for example, see: Sayre et al., (2001), Curr. Med. Chem, 8(7), 721-38; Bains et al., (1997), Brain Res. Rev., 25, 335-358; Alexi et al. (2000), Progress in Neurobiol., 60, 409-470).

Oxidative stress occurs when the normal balance between oxidative events and antioxidative defence mechanisms is disrupted, either by the loss of reducing agents and/or antioxidants or by increased levels of oxidant species. Oxidative stress has been attributed to the actions of highly toxic free-radicals, including reactive oxide species (ROS) such as the superoxide anion ($*O_2^-$) and hydroxyl radical (*OH), and reactive nitrogen species (RNS) derived from nitric oxide (NO) reaction with superoxide or peroxide, such as peroxinitrite ($*ONOO^-$).

Excitotoxic cell death is caused by excessive activation of glutamate receptors by glutamate and glutamatergic agonists such as NMDA and other excitatory amino acids (EAAs). A number of studies also suggests that oxidative stress may act as a mediator in excitotoxically induced neuronal cell death. For example, it has been shown for both NMDA and kainate (a non-NMDA receptor agonist) that activation of EAA receptors increases free-radical damage to lipids, and that this damage can be prevented by simultaneous treatment with antioxidants.

Metabolic compromise may be caused by stroke, asphyxiation, hypoglycaemia and certain poisons interfering with mitochondrial respiration. Mitochondrial dysfunction and resulting depletion of ATP and loss of intracellular calcium buffering capacity can cause an increase in the production of reactive oxygen and nitrogen free-radicals, leading to oxidative stress.

Thus, not only is oxidative stress by free-radicals understood to be a primary factor of neuronal cell death in a number of chronic neurodegenerative diseases, but it may also mediate excitotoxic stimuli and metabolic compromise. Furthermore, the reverse interaction may also occur, as oxidative stress by free-radicals may initiate excitotoxic pathways and cause metabolic impairment.

We have now found the compound L-arginyl-3,4-spermidine, previously disclosed in WO99/31049 for treating of neuronal damage caused by acute ischaemic events such as stroke and head injury, may be used to treat chronic neurodegenerative diseases or conditions in which damage is caused or mediated by free radicals and by excitotoxicity, such as Alzheimer's Disease (AD), Parkinson's Disease (PD), Huntington's Chorea (HC), Multiple Sclerosis (MS) and Amyotrophic Lateral Sclerosis (ALS).

More specifically, we have found, surprisingly, that the compound L-arginyl-3,4-spermidine, previously disclosed in WO99/31049, is neuroprotective against free-radical-induced neuronal cell death in an *in vitro* model of free-radical-induced injury, and is neuroprotective against EAA mediated cell death in an *in vitro* model of excitotoxic injury.

Furthermore, we have shown that L-arginyl-3,4-spermidine is neuroprotective against cell death in an *in vitro* model of ischaemic injury. This *in vitro* model of ischaemic injury (oxygen and glucose deprivation) is even more severe than the *in vitro* hypoxia (oxygen deprivation) and *in vivo* ischaemia models used in WO99/31049. Surprisingly, we have found that L-arginyl-3,4-spermidine is neuroprotective against the much harsher insult of *in vitro* ischaemic injury not only when administered immediately post injury, but even when administration is delayed up to 60 minutes post injury.

In previous studies, as disclosed in WO99/31049, we have found that administration of L-arginyl-3,4-spermidine did not affect blood pressure, heart rate or respiration, or result in adverse motor function.

WO93/12777 and WO91/00853 disclose that polyamine compounds, including a compound (R) referring to racemic arginyl-3,4-spermidine, are useful as calcium ion channel regulators and might therefore have potential uses as prototypic drugs.

Accordingly, in a first aspect, the present invention provides the use of a compound having the general formula (I) or pharmaceutically acceptable salts thereof, for the manufacture of a medicament for treating a chronic neurodegenerative disease or condition:

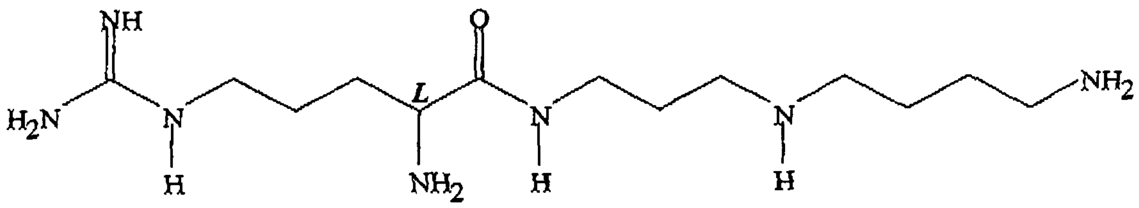

(I)

The invention also provides the use of a compound having the general formula (I) and pharmaceutically acceptable salts thereof for the manufacture of a medicament for treating or protecting from damage or diseases resulting from superoxide production.

The invention also provides the use of a compound having the general formula (1) and pharmaceutically acceptable salts thereof for the manufacture of a medicament for treating or protecting from damage or diseases resulting from peroxynitrite production.

Chronic neurodegenerative diseases or conditions that may be treated in accordance with the invention are those in which neuronal cell death or degeneration is mediated by free-radicals, in particular by ROS including the superoxide anion ($*O_2^-$), whether caused by oxidative stress, excitotoxic stimulus or metabolic dysfunction, or any combination of these factors. Thus, chronic neurodegenerative diseases or conditions treatable in accordance with the invention include AD, PD, HC, MS and ALS, in particular AD, PD and HC.

Without wishing to be bound to a particular mechanism or theory, it is believed that the compound used in accordance with the invention functions as a biological scavenger, by inactivating, consuming, quenching, neutralising or reducing free radicals and ROS.

The compound used according to the present invention is preferably prepared in substantially pure form. By substantially pure is meant a compound which, under conditions of HPLC (high performance liquid chromatography) is not shown to have any or any significant amount of contaminants detectable thereby. Trace levels of contaminants may be acceptable in certain circumstances and such circumstances may be determined by the skilled person at the time. In general, levels of contaminant should be less than 1%, and preferably substantially less than 1%, for example less than 0.1%, possibly as low as 0.001%. In the alternative, it is preferred that the compound is nontoxic, by which is meant that the compound should not exhibit any unacceptable levels of toxicity at the dosages at which it is applied. Preferably, it should exhibit no toxicity whatsoever.

Regardless of the foregoing, the compound defined above is useful in treating chronic neurodegenerative diseases or conditions, based on our tests on the hippocampus, as described below.

The neuroprotective compound herein described may be administered to patients as a prophylactic in order to prevent onset of a chronic neurodegenerative disease. In addition or alternatively, the compound may be applied after onset of a chronic neurodegenerative disease, but it will be appreciated that it is preferred to administer the compound as soon as possible, in order to avoid as much neuronal degeneration as possible. In most circumstances it will be desirable to administer repeated doses at least whilst the patient continues to show symptoms of the disease.

Suitable methods of administration are generally by injection, in order to achieve the desired result as soon as possible, but are not limited to this route. Thus, intravenous injection is particularly preferred but, in some circumstances it may be preferable to administer the compound directly into the cerebrospinal fluid.

The dose of the compound of the present invention will vary depending upon many factors, including the age, body weight and general condition of the patient, as well as the mode, frequency and route of administration. However, a dose of from 0.01 to 50 mg/kg body weight is generally recommended, a dose of from 0.05 to 20 mg/kg body weight being more preferred. This may be administered in a single dose or in divided doses, more preferably in a course of periodic, for example daily, weekly or monthly doses.

Preferably, the compound is used or administered in an amount effective to provide a concentration in the

range of 0.3 to 300 microM, more preferably in the range of 3 to 300 microM.

L-arginyl-3,4-spermidine ("L-Arg3,4"):

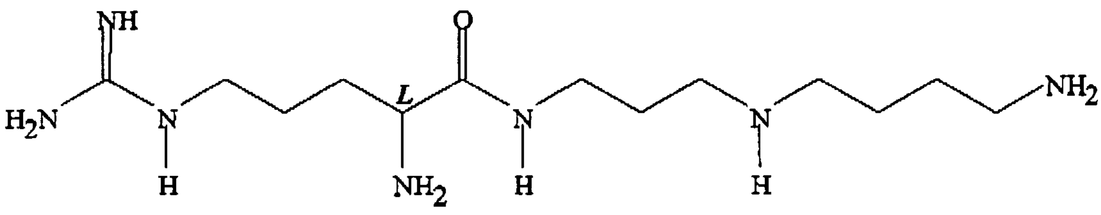

The compound of the present invention may be prepared by a variety of processes which, in themselves, are well-known in the art, for example as disclosed in WO99/31049.

Preparation of an exemplary compound of the invention, as well as neuroprotective activity is illustrated in the accompanying non-limiting examples:

EXAMPLE 1

*Materials*

Drugs: Kainate, NMDA, glutamate, AMPA, and 6,7-Dinitroquinoxaline-2,3(1H,4H)-dione (DNQX) were purchased from Sigma (UK). DNQX was stored at room temperature in DMSO at 40mM. All other drugs were made up as aqueous solutions.

Chemicals and Reagents: Propidium iodide (PI) was purchased form Molecular Probes (Eugene, OR). Dimethyl sulfoxide (DMSO), glucose and glutamine were purchased from Sigma. Millicell CM culture inserts were purchased from Millipore (UK). The 1N-Dde-8N-Mmt-spermidine-4-yl)-carbonyl Wang polystyrene resin was purchased from Novabiochem (Switzerland), and the Fmoc-Arg$(Boc)_2$-OH from Bayer (UK). All other chemicals and reagents were obtained from commercial sources except for L-arginyl-3,4-spermidine ("L-Arg3,4") which was synthesised as indicated below.

*Compound synthesis*

L-Arg3,4 Trifluoroacetic Acid Salt was synthesised as follows:

(1N-1-(4,4-dimethyl-2,6-dioxocyclohexylidene)ethyl-8*N*-monomethoxytrityl-spermidine-4-yl)-carbonyl Wang polystyrene resin (0.279 g, loading 0.42 mmol $g^{-1}$, 0.12 mmol) was swollen in *N,N*-dimethylformamide (DMF). The solvent was then drained and 2% hydrazine in DMF (4 ml) was added. The resulting mixture was spun for 5 min. The solution was drained and the hydrazinolysis repeated twice more. The resin was then washed with DMF (x 3), DCM (x 3), and diethyl ether (x 3). A qualitative ninhydrin test was positive.

The resin was then swollen in DMF and the solvent drained. A solution of 9-fluorenylmethoxycarbonyl-arginine-(*tert*-butyloxycarbonyl)$_2$-OH (0.142 g, 0.24 mmol, 0.24 M) and *N*-hydroxybenzotriazole hydrate (0.037 g, 0.24 mmol, 0.24 M) in dichloromethane (DCM) (0.6 ml) and DMF (0.4 ml) were stirred at room temperature for 10 min, diisopropylcarbodiimide (38 $\mu$l, 0.24 mmol) was added and the resulting solution stirred for a further 10 min. The reaction mixture was then added to the damp resin and spun for 2.5 h. The solution was drained and the resin washed with DMF (x 3), DCM (x 3) and diethyl ether (x 3). A qualitative ninhydrin test was negative.

The resin was swollen in DMF and the solvent drained. A 20% piperidine solution in DMF (4 ml) was added and the reaction mixture was spun for 10 min. The solution was drained and the piperidine treatment repeated. The resin was washed with DMF (x 3), DCM (x 3) and diethyl ether (x 3). A quantitative ninhydrin test was positive.

To the dry resin was added a solution of 97.5% trifluoroacetic acid (TFA) and 2.5% triisopropylsilane (4 ml), and the reaction mixture allowed to stand for 3 h. The solvent was then drained and the resin washed with TFA (2 x 1 ml). The combined acid phases were evaporated *in vacuo.* The residue was dissolved in TFA (0.4 ml) and precipitated in ice-cold diethyl ether. The mixture was centrifuged and the solvent removed by decanting. The pellet was washed with diethyl ether, the mixture centrifuged, and the solvent again decanted. The residue was then dried *in vacuo* overnight. The residue was dissolved in TFA (0.4 ml) and the diethyl ether precipitation repeated. The sample was then dissolved in water and acetonitrile and lyophilised. This was repeated once more to afford L-Arg3,4 as a sticky orange-brown solid (0.061 g, 20% L-Arg3,4 by mass). The compound was quantified by $^1H$ nuclear magnetic resonance spectroscopy with phenol as an internal standard.

EXAMPLE 2

Experiments were conducted using L-Arg3,4 as synthesised according to Example 1:

(i) Protocol for preparation of organotypic hippocampal slice cultures:

Organotypic hippocampal slice cultures were prepared according to established methods (Pringle et al. (1997), Brain Res., 755, 36-46). In brief, Wistar rat pups (8-11 days old) were decapitated and the hippocampus rapidly dissected into ice-cold Gey's balanced salt solution (Gibco Life Technologies, Paisley, UK) supplemented with 4.5 mg $ml^{-1}$ glucose. Slices were separated and plated onto Millicell CM culture inserts (4 per well) and maintained at 37°C and 5% $CO_2$ for 14 days. Maintenance medium consisted of 25% heat-inactivated horse serum, 25% Hank's balanced salt solution (HBSS) and 50% minimum essential medium with added Earle's salts (MEM, ICN Biomedicals, Basingstoke, UK) supplemented with 1 mM glutamine and 4.5mg $ml^{-1}$ glucose. Medium was changed every 3-4 days.

(ii) Protocol for studying effect of ischaemic injury:

Experimental ischaemia or oxygen glucose deprivation was performed as described previously (Pringle et al. (1996), Stroke, 27,2124-2130). Briefly, cultures were transferred to serum-free media (SFM, 75% MEM, 25% HBSS supplemented with 1mM glutamine and 4.5 mg $ml^{-1}$ glucose) containing 5 μg $ml^{-1}$ of the fluorescent exclusion dye propidium iodide (PI). Cultures were allowed to equilibrate in SFM for 30 min prior to imaging, with or without L-Arg3,4. PI fluorescence was detected using a Leica inverted microscope as described below. Any cultures in which PI fluorescence was detected at this stage were excluded from further study. Ischaemia was induced by transferring cultures to glucose-free media (MEM with Earl's salts without glucose and supplemented with 1mM glutamine) saturated with 95%$N_2$ and 5%$CO_2$. Culture plates (without lids) were then sealed into an airtight chamber (Billups-Rothenberg, Del Mar, CA) gassed with 95%$N_2$ and 5%$CO_2$ at 10 L $min^{-1}$ for 10 min before being sealed and placed at 37°C for 50 min. At the end of the ischaemia, cultures were returned to normoxic SFM + PI, with or without compound at the indicated concentrations, and placed back in the incubator for 24 h.

(iii) Protocol for studying effect of excitotoxic injury:

Excitotoxic injury was performed as previously described (Pringle et al. (1997), Brain Res., 755, 36-46) with L-Arg3,4 present pre-, during, and post-injury in treated cultures. Cultures were transferred to SFM + PI with or without 300 μM L-Arg3,4 and allowed to equilibrate for 30 min prior to imaging. Any cultures exhibiting PI fluorescence were excluded. The medium was then exchanged for SFM + PI plus a glutamate receptor agonist (either 10 mM glutamate, 10 μM NMDA, 10 μM AMPA, or 10 μM kainate) for 3 h. After exposure, the media was again exchanged for SFM + PI containing 300μM L-Arg3,4. Cultures were then returned to the incubator until they were assessed for cell death in the indicated region of the pyramidal cell layer 24 h post exposure.

(iv) Protocol for studying effect of free-radical injury:

Free-radical mediated injury was performed as described previously with L-Arg3,4 present pre-, during, and post-injury in treated cultures (Wilde et al., (1997, J. Neurochem., 69, 883-886). Briefly, cultures were transferred to SFM + PI with or without 300 μM L-Arg3,4 and allowed to equilibrate for 30 min prior to imaging. Any cultures in which PI fluorescence was detected at this stage were excluded from further study. Duroquinone (DQ) was dissolved in DMSO at a stock concentration of 100 mM which was then diluted 1:1000 in SFM for a final working concentration of 100 μM (final DMSO concentration of 0.1 %). Free-radical injury was initiated by transferring cultures to SFM supplemented with 100 μM duroquinone for 3 h. At the end of the exposure, cultures were returned to SFM + PI, with or without compound, and placed in the incubator for 24 h.

(v) Protocol for assessment of cell death:

Neuronal damage was assessed as described previously (Pringle et al. (1996), Stroke, 27, 2124-2130; Pringle et al. (1997), Brain Res., 755, 36-46). Light transmission images were captured prior to the induction of injury, and PI fluorescence images recorded at the end of the 24 h post-injury recovery period. All images were captured on an inverted Leica DM-IRBE epifluorescence microscope (Milton Keynes, UK) fitted with a 100 W Hg lamp and standard rhodamine optics (excitation 510-560 nm; dichroic mirror 620 nm; emission > 590 nm). Images were acquired with a 5X NA 0.12 lens and a cooled Hamamatsu digital camera and digitised at 12 bit resolution for analysis in OpenLab 2.1 (Improvision, Coventry, UK) running on a Macintosh G4/400. The area of the CA1 or CA3 cell layer was determined from the transmission image, and the area of PI fluorescence in that cell layers was measured using the density slice function within OpenLab. Neuronal damage was expressed as a percentage of the area in which PI fluorescence was detected above threshold within a cell layer divided by the total area of that cell layer. Protection was calculated as the difference between the damage for a given well and the average damage in untreated controls divided by the average damage in untreated controls Groups were compared against corresponding control groups using the two tailed Student's t-test with significance indicated as * for $P < 0.05$, and ** for $P < 0.01$. Data is presented as mean $\pm$ s.e.mean.

(vi) Protocol for studying electrophysiology:

All animal procedures to produce acute hippocampal slices were approved by the Home Office. Male, 250 g, Sprague-Dawley rats were deeply anaesthetised with halothane and decapitated. The brain was quickly removed, and the hippocampi dissected out and then cut into 300 $\mu$m thick transverse slices on a vibratome (Leica, Milton Keynes, UK) in cold cutting solution (in mM: 189 sucrose, 2.5 KCl, 26 $NaHCO_3$, 1.2 $NaH_2PO_4$, 10 glucose, 5 $MgCl_2$, 0.1 $CaCl_2$) equilibrated with 5% $CO_2$, 95% $O_2$. Slices were transferred to aCSF (in mM: 10 glucose, 125 NaCl, 5 KCl, 26 $NaHCO_3$, 1 $MgCl_2$, 1.25 $NaH_2PO_4$, 2 $CaCl_2$, 10 sucrose) at room temperature, bubbled with 5% $CO_2$ and 95% $O_2$, for at least 1 h before use.

Slices were then transferred to a submerged perfusion chamber (2 ml volume) for the electrophysiological recordings and were perfused at 10ml $min^{-1}$ at 25°C with aCSF equilibrated with 5% $CO_2$ and 95% $O_2$, and allowed to equilibrate for 1 h before manipulations were started. Experimental compounds were applied by bath perfusion. L-Arg3,4 was applied at 300 $\mu$M and DNQX was applied at 10 $\mu$M final concentration. DNQX was diluted from a 40 mM stock in DMSO (final DMSO of 0.025%). The Schaffer collaterals of CA3 were stimulated at voltages from 0 to 30 V with a constant voltage stimulator (Digitimer, Hertfordshire, UK) via a twisted pair stimulating electrode of polyimide coated stainless steel wire (Plastics One, Roanoke, VA). Field potential responses were recorded with an Axopatch 200B amplifier (Axon Instruments, Foster City, CA) in the fast current clamp mode via a 2M KCl filled glass electrode (2-5 M$\Omega$) in the CA1 pyramidal cell layer. Responses were filtered at 2 kHz, digitised at 20 kHz with a Digidata 1320A (Axon Instruments), and stored onto hard disk with Clampex 8.1 software (Axon Instruments) running on a Dell Pentium III PC. Data was analysed in Clampfit 8.1 (Axon Instruments).

Stimulus-response (S/R) curves were generated from stimuli from 0 to 30 V in 2 V increments. Bath perfusion of 300 $\mu$M L-Arg3,4 in aCSF was applied for 10 min with a sub-maacimal stimulus applied every minute to monitor synaptic transmission. A second S/R curve was generated before the perfusion was switched back to plain aCSF. A sub-maximal stimulus was applied every minute during the 15 min wash period after which a third S/R curve was generated. Perfusion of 10 $\mu$M DNQX was then started until the response to the sub-maximal stimulus was completely eliminated which always occurred within 10 min. Increasing the stimulus to maximum (30V) did not generate a response, and therefore, no S/R curves were generated in the presence of DNQX. In Clampfit, S/R curves were fit to a sigmoidal function of the form:

$$R = \frac{R_{Max}}{1 + e^{m \bullet (V_{50} - S)}}$$

where R is the magnitude of the evoked response, $R_{max}$ is the maximum response, $V_{50}$ is the voltage which produces a half maximal response, S is the stimulus voltage, and m is proportional to the slope of the linear region of the sigmoid. Data was compared by one way ANOVA.

Hydroethidine (HEt) has been utilised to fluorescently monitor the production of superoxide in primary neuronal cultures [Bindokas *et al.,* (1996), Carriedo *et al.* (1998)]. A stock solution of HEt was made was made in $N_2$ sparged DMSO (10 mg $ml^{-1}$) which was then aliquoted and stored at -80°C. aliquots were used only once before being discarded. Two separate experimental paradigms were employed to test the effect of L-Arg3,4 on superoxide production in response to either AMPA or NMDA stimulation.

For the AMPA experiments, organotypic slice cultures were loaded with 1 $\mu$g $ml^{-1}$ HEt in HEPES buffered salt solution (HSS) (in mM): glucose 10, NaCl 144, KCl 5, HEPES 10, $MgCl_2$ 1, $CaCl_2$ 2, pH 7.4, for 30 minutes in the dark. All subsequent solutions contained HEt to prevent a decrease in signal due to reporter depletion. Images were acquired once per minute for the duration of the experiment on the same system as described above for assessment of cell death. A 10 minute record of baseline superoxide production was produced before the bathing solution was exchanged for HBSS + HEt containing 10 $\mu$M AMPA, again for 10 minutes. As shown previously, stimulation of glutamate receptors with AMPA lead to an increase in superoxide production [Bindokas *et al.,* (1996), Carriedo *et al.* (1998)]. The bathing solution was then exchanged for HBSS + HEt containing both 10 $\mu$M AMPA and 300 $\mu$M L-Arg3,4 for 10 minutes to test the effect of L-Arg3,4 on superoxide production. In separate control experiments, 300 $\mu$M L-Arg3,4 in HSS was incubated

with slices to determine if it affected baseline superoxide production on its own.

Acquired images from the AMPA experiments were then analysed with the OpenLab image analysis package. A region of interest was drawn around the CA1 pyramidal cell layer because this was the cell layer that produced the largest increase in fluorescence in response to AMPA activation. In each image, within the region of interest, the average pixel value was calculated in arbitrary units (AU) to produce a record of fluorescence intensity values over time. A linear regression was then performed on the data to calculate the rate of superoxide production as change in intensity over time (arbitrary units per minute, AU $min^{-1}$) for each of the treatments. Data is presented as mean ± s.d. Rates of superoxide production were then compared by ANOVA followed by *post-hoc* comparisons with Bonferroni corrections with significance indicated as * for $P$<0.05 and * * for $P$<0.01.

RESULTS (Example 2):

The results are depicted in Figures 2 to 7.

<u>(i) Effect of L-Arg3,4 on ischaemic cell death:</u>

One hour of ischaemia generated reproducible neurodegeneration in the pyramidal cell layer in hippocampal slice cultures of approximately 38 ± 2% as determined by PI staining. Cell death in untreated control cultures from individual ischaemia experiments was not statistically different ($P$ = 0.70), and, therefore, ischaemic controls were combined into a single group (Figure 2: "Control") (n =113). Incubation of cultures in 300 μM L-Arg3,4 pre-, during, and post-ischaemia resulted in a significant reduction of neurodegeneration (Figure 2: "PDP") to 23 ± 4% (39% protection, $P$ < 0.01, n = 34).

<u>(ii) Effect of L-Arg3,4 on ischaemic cell death when administration is delayed:</u>

To determine the effective time window after injury, administration of L-Arg3,4 was delayed until after the completion of the ischaemia and applied at 0, 10, 30, and 60 min post-injury. Delayed application of 300 μM L-Arg3,4 significantly decreased cell death as compared to controls at 0, 10, and 60 min post-injury (Figure 2) reducing damage to 23 ± 3% (40% protection, $P$ < 0.01, n = 36), 15 ± 2% (59% protection, $P$ < 0.01, n = 24), and 26 ± 3% (30% protection, $P$ < 0.05, n = 31), respectively. Although damage was reduced to 28 ± 4% at 30 min post injury, the 26% protection did not reach statistical significance ($P$ < 0.08, n = 25).

<u>(iii) Effect of varying dose of L-Arg3,4 on ischaemic cell death:</u>

The effective post-injury dose for neuroprotection against ischaemia was tested with concentrations of L-Arg3,4 ranging from 300 nM to 300 μM. A dose of 300 μM was initially chosen for all studies based on the data above in a model of hypoxic injury which indicated that 300 μM was an optimal dose. Untreated controls from separate experiments were not significantly different ($P$ = 0.9) with an aggregate damage of 34 ± 9% and were combined into a single group (Figure 3: "Control") ($n$ = 47). At 300 nM, L-Arg3,4 did not reduce damage, however, at 3, 30, and 300 μM, the compound did reduce damage to 19 ± 5% (43% protection, $P$ < 0.06, n = 14), 11 ± 3% (67% protection, $P$ < 0.05, n = 8), and 23 ± 3% (32% protection, $P$ < 0.05, n=36), respectively as compared to the untreated group (Figure 3).

<u>(iv) Effect of L-Arg3,4 on excitotoxic cell death:</u>

Administration of 300 μM L-Arg3,4 significantly reduced cell death in the CA1 cell layer generated by 10 mM Glut (Figure 4) from 21 ± 3% damage in controls (n = 23) to 13 ± 3% in treated cultures (41% protection, $P$ < 0.05, n = 23) suggesting that it could be a glutamate receptor antagonist. To determine if its actions were glutamate receptor subtype specific, L-Arg3,4 was tested against 10 μM NMDA, 10 μM AMPA, and 10 μM kainate in separate experiments which produced regionally specific cell death. Administration of 300 μM L-Arg3,4 significantly reduced the cell death in CA1 generated by 10 μM NMDA (Figure 4) from 25 ± 3% damage in controls ($n$ = 42) to 16 ± 2% in treated cultures (38% protection, $P$ < 0.05, n = 43) or 10 μM AMPA (Figure 4) from 41 ± 5% damage in controls ($n$ = 21) to 19 ± 4% in treated cultures (53% protection, $P$ < 0.01, n = 22). Furthermore, 300 μM L-Arg3,4 significantly reduced the cell death in CA3 due to 10 μM kainate (Figure4: "KA") from 27 ± 6% damage in controls ($n$ = 21) to 11 ± 4% in treated cultures (61 % protection, $P$ < 0.05, n = 24).

<u>(v) Effect of L-Arg3,4 on synaptic transmission:</u>

Bath application of 300 μM L-Arg3,4 did not grossly affect evoked responses from acute hippocampal slices.

Individual evoked responses from a single slice before, during, and after L-Arg3,4 application showed that the compound had no effect on evoked responses (Figure 5A). This lack of effect was in contrast to the effect of 10 μM DNQX which blocked the evoked response completely (Figure 5B). The population spike of a single slice to a sub-maximal stimulus was not affected by L-Arg3,4 infusion nor its wash out. However, in the same slice, 10 μM DNQX eliminated all transmission within 5 minutes (Figure 5B). S/R curves for a slice before, during, and after L-Arg3,4 were not affected by bath application of 300μM L-Arg3,4 (Figure 5C). All S/R curves were fitted to a sigmoidal function so that comparisons between aggregate S/R curves could be made (Table 1). None of the parameters was significantly affected by L-Arg3,4 infusion as determined by a one way ANOVA for each parameter.

Table 1

| | $R_{max}$(mV) | $V_{50}$ (V) | m | n |
|---|---|---|---|---|
| **Pre** | 2.61±1.16 | 19.28±6.02 | 0.32±0.18 | 6 |
| **Arg-3,4** | 2.71±1.31 | 15.98±5.12 | 0.59±0.48 | 6 |
| **Wash** | 2.38 ±0.83 | 15.07 ± 5.39 | 0.78 ± 0.60 | 3 |
| ***P*** | 0.92 | 0.48 | 0.29 | |

(vi) Effect of L-Arg3,4 on free-radical mediated cell death:

Administration of 300 μM L-Arg3,4 significantly reduced cell death in the CA1 pyramidal cell layer caused by a 3 h incubation in 100 μM duroquinone (Figure 6), indicating that it is quenching free-radicals. Injury was reduced from 40 ± 5% in untreated controls ($n$ = 21) to 21 ± 5% in treated cultures (protection 46%, $P$ < 0.05, $n$ = 21).

In addition to blocking free-radical mediated damage, we have also demonstrated that L-Arg3,4 significantly reduces the production of superoxide radicals by AMPA implying that the compound has a direct effect on free-radical production.

(vii) Effect of L-Arg3,4 on superoxide production)

Production of superoxide was reliably measured fluorescently with HEt as described by Bindokas *et al.* (1996) and Carriedo *et al.* (1998). In the AMPA stimulation experiments, baseline superoxide production was measured to be 2.06 ± 1.60 AU $min^{-1}$ ($n$=16) and was not affected by 300 μM L-Arg3,4, 2.55 ± 2.02 AU $min^{-1}$ ($P$>0.6, data not shown). Stimulation of glutamate receptors by 10 μM AMPA significantly increased the rate of superoxide production approximately 5 fold to 10.65 ± 3.70 AU $min^{-1}$ ($n$=18, $P$<0.001, Figure 7). The subsequent addition of 300 μM L-Arg3,4, in the continued presence of 10 μM AMPA, significantly reduced the rate of superoxide production by approximately half to 5.07 ± 1.94 AU $min^{-1}$ compared to the rate with AMPA alone ($n$=4, $P$<0.01), although this rate was still significantly elevated from baseline ($P$<0.01).

DISCUSSION OF RESULTS (Example 2):

Organotypic hippocampal slice culture models are ideal preparations for the search of novel drugs because they allow for the rapid screening of a large number of compounds. These culture preparations possess the added advantage over other *in vitro* systems of maintaining several key similarities to *in vivo* models including delayed neurodegeneration and selective vulnerability of either the CA1 or CA3 pyramidal cell layer depending on the injury paradigm and severity.

We have extended previous results obtained with a model of hypoxia to a more severe *in vitro* injury paradigm of ischaemia (oxygen glucose deprivation) and have shown that L-Arg3,4 significantly prevented CA1 cell loss after 1 h of ischaemia (Figure 2). To determine the available therapeutic window for L-Arg3,4, administration was delayed for up to 60 min post-ischaemia and, surprisingly, it was still neuroprotective. This therapeutic window is over twice as long as that for MK-801, D-APV, or dextromethorphan which lose effectiveness if administration is delayed for 30 min in models of excitotoxicity (Hartley & Choi, (1989), J Pharmacol. Exp. Ther., 250, 752-758), suggesting that the mechanism of action of L-Arg3,4 is unlikely to be at the level of the NMDA receptor.

Given the well established involvement of glutamate in the pathophysiological sequelae of ischaemia, we tested whether L-Arg3,4 was neuroprotective against EAA mediated cell death. L-Arg3,4 significantly prevented cell death due to glutamate, NMDA, AMPA, and kainate toxicity.

Given its structural similarity to spermidine (Figure 1(i)), L-Arg3,4 could be acting at the polyamine site of the NMDA receptor. L-Arg3,4 (Figure 1(ii)) does not potentiate NMDA receptor activation because it did not exacerbate NMDA mediated neurotoxicity in our studies, and therefore, probably does not interact with the polyamine site on the NMDA receptor.

In our experiments, L-Arg3,4 did not affect synaptic transmission in any way as determined by measuring evoked field responses in acute hippocampal slices (Figure 5) reaffirming that it does not interact with voltage sensitive calcium channels (VSCC). This lack of effect was in sharp contrast to the effect of 10 μM DNQX which abolished all synaptic transmission within 10 min of exposure indicating that L-Arg3,4 does not alter transmission mediated by non-NMDA receptors.

Its lack of electrophysiological effects in combination with its ability to prevent ischaemic and excitotoxic cell death by both NMDA and non-NMDA receptor agonists suggests that L-Arg3,4 acts down stream of receptor and or channel activation. Its very long therapeutic window also suggests that it may target a nexus of the pathological sequelae which is common to these models of cell death especially since NMDA antagonists have a much shorter therapeutic window of less than 30 min in models of excitotoxicity (Hartley & Choi, (1989), J Pharmacol. Exp. Ther., 250, 752-758).

To test the hypothesis that the protection afforded by L-Arg3,4 was due to its ability to react with ROS, cultures were injured by incubation in 100 μM duroquinone which specifically generates superoxide by disruption of the mitochondria electron transport chain (Wilde et al., (1997), J. Neurochem., 69, 883-886). Surprisingly, L-Arg3,4 significantly protected against free-radical mediated injury suggesting that it can quench ROS (Figure 6).

In summary, we have studied the neuroprotective mechanism of action of L-Arg3,4, which prevents cell death in an *in vitro* ischaemia model. Surprisingly, delayed administration of L-Arg3,4 after ischaemia (up to 60 min) was also neuroprotective. This extremely long therapeutic window *in vitro* is in contrast to NMDA antagonists, such as MK-801, which lose efficacy as early as 15 min post-injury (Hartley & Choi, (1989), J Pharmacol. Exp. Ther., 250, 752-758). The compound prevented cell death due to glutamate but was not receptor sub-type specific as it also prevented cell death due to NMDA, AMPA, and kainate. The compound did not inhibit synaptic transmission, and therefore, was not likely to directly antagonise glutamate receptors or VSCC unlike a structurally similar compound, sFTX-3.3 (Figure 1(iii)). L-Arg3,4 also prevented superoxide mediated cell death. Because it did not affect synaptic transmission, L-Arg3,4 may engender fewer detrimental side effects than previous, experimental, therapeutic compounds.

## Claims

1. The use of a compound having the general formula (I) or pharmaceutically acceptable salts thereof, for the manufacture of a medicament for treating a chronic neurodegenerative disease or condition:

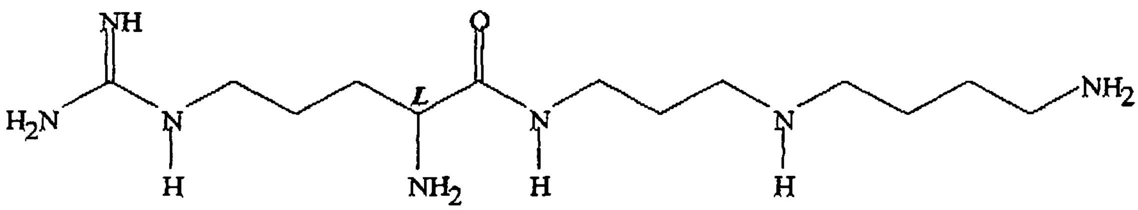

(I)

2. The use according to claim 1 wherein the chronic neurodegenerative disease or condition is selected from Alzheimer's Disease, Parkinson's Disease, Huntington's Chorea, Multiple Sclerosis and Amyotrophic Lateral Sclerosis.

3. The use according to claim 1 or claim 2 wherein the compound is used or administered in an amount effective to provide a concentration in the range of 0.3 to 300 μM.

4. The use according to claim 3 wherein the compound is used or administered in an amount effective to provide a concentration in the range of 3 to 300 μM.

5. The use of a compound according to claim 1 or claim 2 for the manufacture of a medicament for treating or protecting from damage or diseases resulting from superoxide production.

6. The use of a compound according to claim 1 or claim 2 for the manufacture of a medicament for treating or protecting from damage or diseases resulting from peroxynitrite production.

**Patentansprüche**

1. Verwendung einer Verbindung mit der allgemeinen Formel (I) oder pharmazeutisch verträglicher Salze davon für die Herstellung eines Medikaments zum Behandeln einer chronischen neurodegenerativen Krankheit oder eines Zustands:

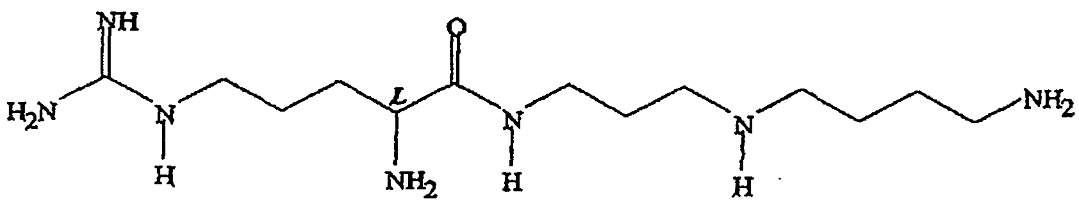

(I)

2. Verwendung nach Anspruch 1, wobei die chronische neurodegenerative Krankheit oder der Zustand aus Alzheimer-Krankheit, Parkinson-Krankheit, Chorea Huntington, Multipler Sklerose und Amyotropher Lateralsklerose ausgewählt ist.

3. Verwendung nach Anspruch 1 oder Anspruch 2, wobei die Verbindung in einer Menge, wirksam, um eine Konzentration in dem Bereich von 0,3 bis 300 μM bereitzustellen, verwendet oder verabreicht wird.

4. Verwendung nach Anspruch 3, wobei die Verbindung in einer Menge, wirksam, um eine Konzentration in dem Bereich von 3 bis 300 μM bereitzustellen, verwendet oder verabreicht wird.

5. Verwendung einer Verbindung nach Anspruch 1 oder Anspruch 2 für die Herstellung eines Medikaments zum Behandeln oder Schützen vor Schädigung oder Krankheiten, resultierend aus Superoxidproduktion.

6. Verwendung einer Verbindung nach Anspruch 1 oder Anspruch 2 für die Herstellung eines Medikaments zum Behandeln oder Schützen vor Schädigung oder Krankheiten, resultierend aus Peroxynitritproduktion.

**Revendications**

1. Utilisation d'un composé de formule générale (I) ou d'un de ses sels pharmaceutiquement acceptables, pour la fabrication d'un médicament indiqué pour le traitement d'une maladie ou d'un état neurodégénératif chronique:

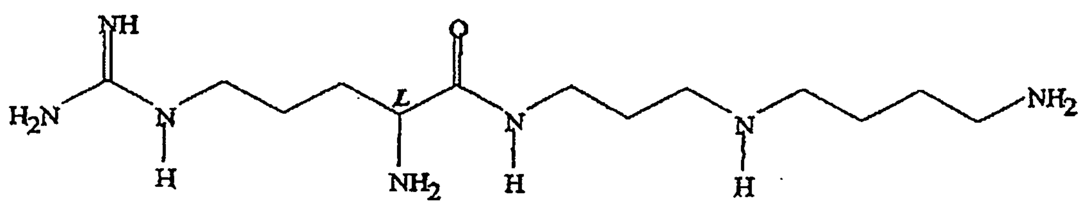

(I)

2. Utilisation selon la revendication 1 dans laquelle la maladie ou l'état neurodégénératif chronique est choisi parmi la maladie d'Alzheimer, la maladie de Parkinson, la chorée de Huntington, la sclérose en plaques, et la sclérose latérale amyotrophique.

3. Utilisation selon la revendication 1 ou la revendication 2 dans laquelle le composé est utilisé ou administré en une quantité efficace pour donner une concentration dans la gamme de 0,3 à 300 μM.

**4.** Utilisation selon la revendication 3 dans laquelle le composé est utilisé ou administré en une quantité efficace pour donner une concentration dans la gamme de 3 à 300 μM.

**5.** Utilisation d'un composé selon la revendication 1 ou la revendication 2 pour la fabrication d'un médicament indiqué pour le traitement ou la protection contre des dommages ou des maladies résultant de la production de superoxyde.

**6.** Utilisation d'un composé selon la revendication 1 ou la revendication 2 pour la fabrication d'un médicament indiqué pour le traitement ou la protection contre des dommages ou des maladies résultant de la production de peroxynitrite.

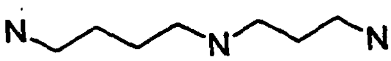

(i)

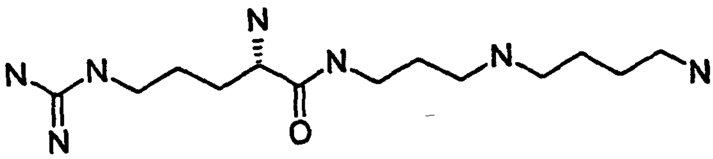

(ii)

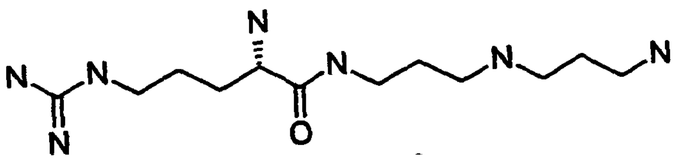

(iii)

Fig. 1

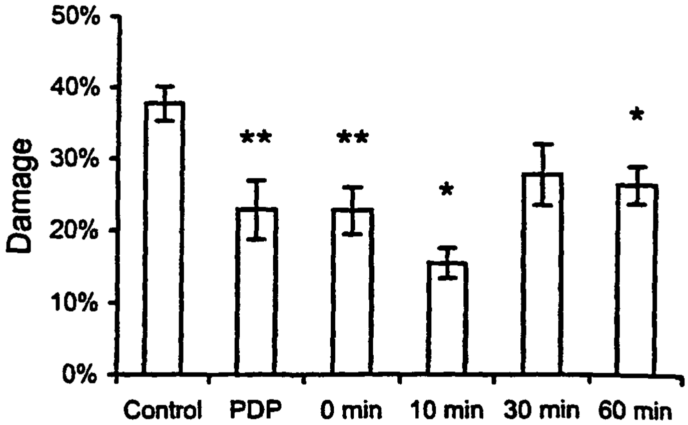


Fig. 2

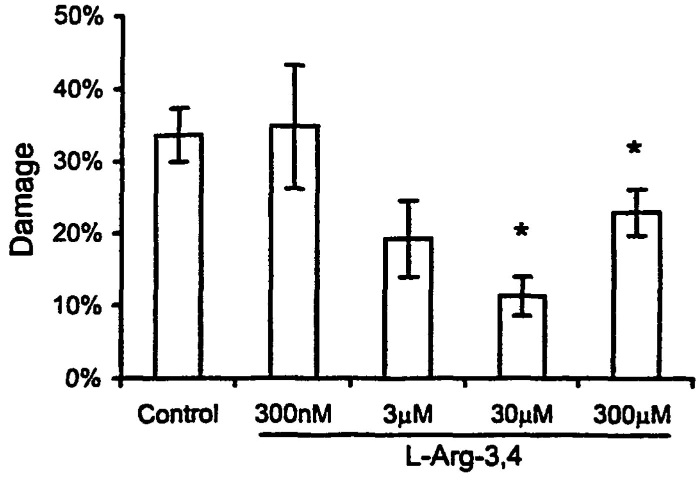


Fig.3

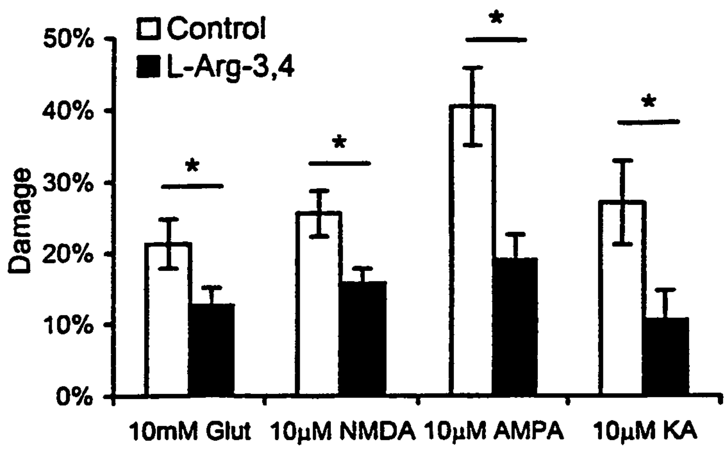

Control
L-Arg-3,4
50%
40%
30%
20%
10%
0%
Damage
*
10mM Glut
10μM NMDA
10μM AMPA
10μM KA


Fig. 4

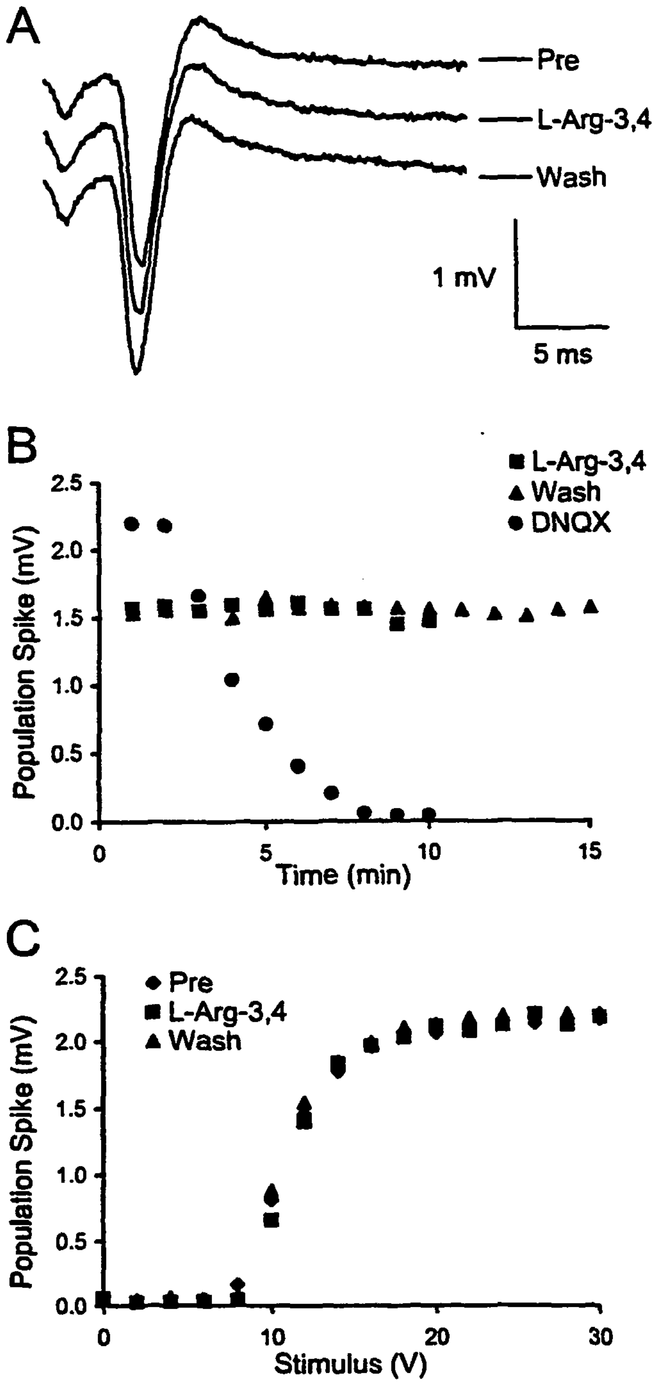
A
Pre
L-Arg-3,4
Wash
1 mV
5 ms
B
L-Arg-3,4
Wash
DNQX
Population Spike (mV)
2.5
2.0
1.5
1.0
0.5
0.0
0
5
10
15
Time (min)
C
Pre
L-Arg-3,4
Wash
Population Spike (mV)
2.5
2.0
1.5
1.0
0.5
0.0
0
10
20
30
Stimulus (V)

Fig. 5

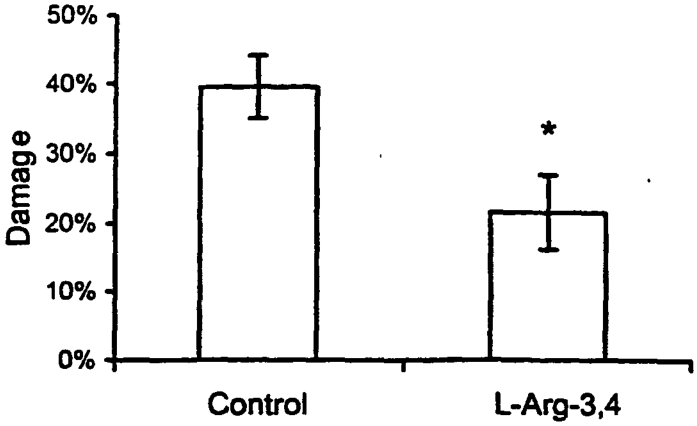
50%
40%
30%
20%
10%
0%
Damage
*
Control
L-Arg-3,4

Fig. 6

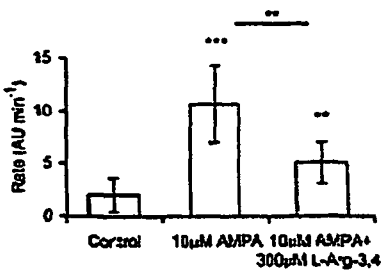
15
10
5
0
Rate (AU min-1)
***
**
**
Control
10μM AMPA
10μM AMPA+ 300μM L-Arg-3,4

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9931049 A
- WO 9312777 A
- WO 9100853 A


### Non-patent literature cited in the description


- **SAYRE et al.** *Curr. Med. Chem,* 2001, vol. 8 (7), 721-38
- **BAINS et al.** *Brain Res. Rev.,* 1997, vol. 25, 335-358
- **ALEXI et al.** *Progress in Neurobiol.,* 2000, vol. 60, 409-470
- **PRINGLE et al.** *Brain Res.,* 1997, vol. 755, 36-46
- **PRINGLE et al.** *Stroke,* 1996, vol. 27, 2124-2130
- **WILDE et al.** *J. Neurochem.,* 1997, vol. 69, 883-886
- **HARTLEY ; CHOI.** *J Pharmacol. Exp. Ther.,* 1989, vol. 250, 752-758